Europäisches Patentamt

(19) European Patent Office

Office européen des brevets

(11) Publication number: **0 237 348**
**B1**

## (12) EUROPEAN PATENT SPECIFICATION

(45) Date of publication of the patent specification:
05.09.90

(51) Int. Cl.⁵: **A61K 31/52**

(21) Application number: 87302132.3

(22) Date of filing: 12.03.87

(54) Cell protection.

(30) Priority: 13.03.86  GB 8606283
03.10.86  GB 8623835

(43) Date of publication of application:
16.09.87 Bulletin 87/38

(45) Publication of the grant of the patent:
05.09.90 Bulletin 90/36

(84) Designated Contracting States:
BE CH DE FR GB IT LI LU NL SE

(56) References cited:
WO-A-86/01110
US-A- 3 624 205

P.H. LIST et al.: "HAGERS HANDBUCH DER
PHARMAZEUTISCHEN PRAXIS", 4th edition, 1977,
page 358, Springer Verlag, Berlin, DE

(73) Proprietor: THE WELLCOME FOUNDATION LIMITED,
Unicorn House 160 Euston Road, London NW1 2BP(GB)

(72) Inventor: Namm, Donald H., 1313 Ravenhurst Drive,
Raleigh, NC 27609(US)
Inventor: Spector, Thomas, Route 7 Box 59, Durham,
NC 27707(US)

(74) Representative: Rollins, Anthony John et al, Group
Patents & Agreements The Wellcome Research
Laboratories Langley Court, Beckenham Kent
BR3 3BS(GB)

ACTORUM AG

## Description

The present invention relates to the reduction of cellular damage following an ischemic episode and to pharmaceutical formulations useful in such reduction.

Ischemic heart disease is the single most frequent cause of death in the U.S.A. It results from the obstruction of blood flow in one or more of the coronary arteries (which supply blood to heart muscle). A heart attack (acute myocardial infarction) occurs when a coronary artery is blocked completely. This can lead to death from arrhythmias or reduced contractile function of the heart.

Paradoxically, reestablishment of blood flow after a period of ischemia causes further damage to the heart muscle served by the previously obstructed coronary artery. This reperfusion injury is caused in part by oxygen free radicals produced when molecular oxygen is reintroduced to the tissue.

In patent application WO 8 601 110 it is suggested that the enzyme xanthine oxidase is the primary source of superoxide (an oxygen free radical) which mediates reperfusion damage, and that inhibitors of xanthine oxidase, such as allopurinol or oxypurinol can protect against such damage.

Allopurinol has been widely presented for the treatment of gout for over twenty years. Oxypurinol, (4,6-dihydroxypyrazola(3,4-d)pyrimidine) the major metabolite of allopurinol, has occasionally been used for such treatment in patients allergic to allopurinol.

We have now discovered that whilst both allopurinol and oxypurinol are effective in reducing cellular injury when administered prior to ischemia, only oxypurinol is effective when administered after ischemia (and before reperfusion). In addition, contrary to the teaching of patent application WO 8 601 110 it has been found that the intravenous route of administration is preferred, and indeed it would not be possible to deliver an effective dose by the intramuscular route.

In a first aspect, the present invention provides the use of oxypurinol for the manufacture of a medicament for use in the reduction of cellular damage following ischemia and prior to reperfusion.

In a second aspect, the present invention provides oxypurinol for use in the manufacture of a medicament to prevent cellular damage following ischemia and prior to reperfusion.

Oxypurinol is normally administered intravenously for use in accordance with the present invention.

The amount of oxypurinol required to protect cells from further damage following ischemia will vary with the condition of the patient. However, for a human being a suitable intravenous dose of oxypurinol is in the range of from 0.5 mg/kg to 15 mg/kg per day and preferably in the range of from 1 mg/kg to 10 mg/kg per day, for example, 5 mg/kg. Oxypurinol will normally be administered up to one hour, for example up to 45 minutes, before reperfusion.

Oxypurinol can be used in combination with or as a complement to other therapeutic agents and procedures. For example, oxypurinol can be used with thrombolytic agents (e.g., tissue activator (t-PA), streptokinase, urokinase, apsac (Eminase®) and other such agents), agents which treat vascular spasm, mechanical methods for revascularization of ischemic tissue, agents which preserve purines such as adenosine deaminase inhibitors, free radical scavengers such as superoxide dismutase, and agents which salvage ischemic tissue such as t-PA.

Intravenous formulations of oxypurinol will comprise a solubilized form of the active compound, such as a salt, conveniently an alkali metal salt or quartenary ammonium salt, together with one or more pharmaceutically acceptable carriers thereof and optionally any other therapeutic ingredients. The carrier(s) must be pharmaceutically acceptable in the sense of being compatible with the ether ingredients of the formulation and not deleterious to the recipient thereof.

Intravenous formulations containing the sodium salt of oxypurinol are preferred. The formulations may conveniently be presented in unit dosage form and may be prepared by any of the methods well known in the art of pharmacy. All methods include the step of bringing the active compound into association with the liquid carrier with heating if necessary.

Formulations suitable for intravenous administration include aqueous and nonaqueous sterile injection solutions which may contain, in addition to the active ingredient, antioxidants, buffers, bacteriostats, isotonic agents and other ingredients to promote bioavailability. Oxypurinol is conveniently present in salt form in such formulations.

For convenience, an injectable formulation containing oxypurinol can be premeasured and preloaded into a disposable syringe and the loaded syringe sterilized and packaged in a sterile container. Such a packaging form would be especially useful to emergency medical personnel since time of treatment is critical after an ischemic attack.

In addition to the aforementioned ingredients, the formulations of this invention may further include one or more accessory ingredient(s) selected from diluents, buffers, surface active agents, preservatives (including antioxidants).

Example 1
Injectable (i.v.) Formulation

| Ingredients | mg/vial |
|---|---|
| Oxypurinol | 150.0 |
| Sodium Hydroxide, 1N | qs to pH 12.0 |
| Water for Injection | qs to 15.0 ml |

The oxypurinol is added to a solution of sodium hydroxide in 10 ml of water for injection (USP). The pH of the solution is adjusted to 12.0 using sodium hydroxide (1N). After adding additional water for injection, the solution is filtered, sterilized and placed in sterilized vials. The solution is lyophilized to form a sterile, dry cake, and the vial is sealed under sterile conditions. The solution is reconstituted with water for injection just prior to administration to the patient.

Example 2
Injectable (i.v.) Formulation

| Ingredients | mg/vial |
|---|---|
| Oxypurinol | 350 mg |
| Sodium Hydroxide 0.1N | qs to pH 11.5 |
| Water for Injection | qs to 25 ml |

The oxypurinol is added to a solution of sodium hydroxide in 23 ml of water for injection (USP). The pH of the solution is adjusted to 11.5 using sodium hydroxide solution (0.1N). The total volume of the solution is brought to 25 ml by addition of water for injection. The solution is filtered through a sterilizing membrane, aseptically filled, and lyophilized until dry. The vial is sealed under sterile conditions. The solution is reconstituted with water for injection just prior to administration to the patient.

Example 1

Methodology

Male beagle dogs (10–12 kg) were anaesthetized with pentobarbital sodium, intubated, and ventilated with room air via a Harvard respirator. Catheters for infusion and arterial blood pressure measurements were implanted in the left jugular vein and left carotial artery. A thoracotomy was performed at the fourth intercostal space, the heart suspended in a percardial cradle and the left anterior descending artery (LAD) isolated just below the first major diagonal branch. An electromagnetic flow probe was placed on LAD. A 90 minute occlusion of the LAD was produced by placing a snare of 1/0 silk sutre distel to the flow probe. Treatment was initiated intravenously fifteen minutes prior to release of this snare occlusion and continued for 45 minutes after release. The thoracotomy was closed, and the animals were allowed to recover from the surgical procedures. The animals were reanaesthetized 24 hours after the occlusion, and the flow in the LAD reassessed. Then the heart was removed for post mortem quantification of infarct size.

Three groups of dogs were evaluated. Group 1 consisted of saline controls. Group 2 were administered allopurinol and group 3 were administered oxypurinol. Allopurinol and ozypurinol were dissolved, separately, in O.I.N. sodium hydroxide and back-titrated to pH 9.3. The formulations were adminstered intravenously over five minutes beginning fifteen minutes prior to reperfusion.

Myocaridal infarct site was quantified by an ex vivo dual reperfusion technique. Cannulas were inserted into the LAD immediately distel to the site of occlusion and into the aorta above the coronary ostia. The LAD coronary bed that was perfused with 1.5% triphenyl tetrazolium hydrochloride (TTC) in 0.02 M potassium phosphate buffer, pH 7.4. The aorta was perfused in a retrograde manner with 0.5% Evans blue dye. Both regions were perfused with their respective stains at a constant pressure of 100 mm mercury for five minutes. The heart was cut into 8 mm slices perpendicular to the apex-base axis. The area of the left ventrical at risk of infarction due to aatomical dependence on the LAD for blood flow is identified by the lack of Evans blue in this region. The region of infarcted myocardium within the area at risk was demarcated by the lack of staining of the tissue when perfused with TTC due to a loss of dehydrogenase enzymes.

The transverse ventricular sections were traced carefully on to clear acrylic overlays to provide a permanent record of infarcted methology and to allow planimetric confirmation of infarct size. Ventricular sections then were trimmed of right ventricular muscle, valvular, and fatty tissue. Total left ventricular, area at risk, and infarct was separated by careful dissection and weighted. Infarct size was expressed as a percentage of the anatomic area at risk. Statistical comparsion of the drug treatment group to the control group were made using a one way analysis of variants (anova) using Bonferroni's method for multiple comparison (Circulation Research, 1980, 47, 1–9). A p value of less than 0.05 was taken as the criterion of significance.

Results

TABLE

| Treatment | Numer of dogs | % Area at Risk * Infarcted |
|---|---|---|
| Saline | 6 | 37.3 ± 3.5 |
| Allopurinol, 10 mg/kg | 5 | 43.1 ± 6.1 |
| Oxypurinol, 10 mg/kg | 6 | 19.0 ± 1.7 |

* Data are expressed as means ± standard errors.

Conclusion
Oxypurinol significantly reduced the area at risk compared with saline and allopurinol.

**Claims**

1. Use of oxypurinol for the manufacture of a medicament for use in the reduction of cellular damage following ischemia and prior to reperfusion.
2. Use of oxypurinol for the manufacture of a medicament for administration to a patient following ischemia and prior to reperfusion.
3. Use of oxypurinol for the manufacture of a medicament for prevention of cellular damage following ischemia and prior to reperfusion.
4. Use of oxypurinol according to any one of claims 1 to 3 in the form of a formulation suitable for intravenous administration.
5. Use of oxypurinol according to claim 4 wherein the oxypurinol is present in the form of an alkali metal salt.
6. Use of oxypurinol according to claim 5 wherein the salt is the sodium salt of oxypurinol.
7. Use according to claim 6 wherein the medicament is an intravenous formulation of the sodium salt oxypurinol for administration to a patient at a dose of about 5 mg of oxypurinol per 1 kg body weight of the patient.
8. Use of oxypurinol according to any one of claims 1–4 in the form of the sodium salt.

**Patentansprüche**

1. Verwendung von Oxypurinol zur Herstellung eines Arzneimittels zur Verminderung von Zellschäden nach der Ischämie und vor der Reperfusion.
2. Verwendung von Oxypurinol zur Herstellung eines Arzneimittels zur Verabreichung an einen Patienten nach der Ischämie und vor der Reperfusion.
3. Verwendung von Oxypurinol zur Herstellung eines Arzneimittels zur Verhinderung von Zellschäden nach der Ischämie und vor der Reperfusion.
4. Verwendung von Oxypurinol nach einem der Ansprüche 1 bis 3 in Form einer für die intravenöse Verabreichung geeigneten Formulierung.
5. Verwendung von Oxypurinol nach Anspruch 4, worin das Oxypurinol in Form eines Alkalimetallsalzes eingesetzt wird.
6. Verwendung von Oxypurinol nach Anspruch 5, worin als Salz das Natriumsalz von Oxypurinol verwendet wird.
7. Verwendung nach Anspruch 6, worin das Arzneimittel eine zur intravenösen Verabreichung geeignete Formulierung des Natriumsalzes von Oxypurinol zur Verabreichung eines Dosis von etwa 5 mg Oxypurinol pro kg Körpergewicht des Patienten vorliegt.
8. Verwendung von Oxypurinol nach einem der Ansprüche 1 bis 4 in Form des Natriumsalzes.

## Revendications

1. Utilisation de l'oxypurinol pour la production d'un médicament à utiliser dans la réduction des lésions cellulaires après l'ischémie et avant la reperfusion.

2. Utilisation de l'oxypurinol pour la production d'un médicament à administrer à un patient après l'ischémie et avant la reperfusion.

3. Utilisation de l'oxypurinol pour la production d'un médicament pour la prévention des lésions cellulaires après l'ischémie et avant la reperfusion.

4. Utilisation de l'oxypurinol suivant l'une quelconque des revendications 1 à 3, sous la forme d'une composition se prêtant à l'administration intraveineuse.

5. Utilisation de l'oxypurinol suivant la revendication 4, dans laquelle l'oxypurinol est présent sous la forme d'un sel de métal alcalin.

6. Utilisation de l'oxypurinol suivant la revendication 5, dans laquelle le sel est le sel de sodium de l'oxypurinol.

7. Utilisation suivant la revendication 6, dans laquelle le médicament est une composition intraveineuse du sel de sodium de l'oxypurinol pour l'administration à un patient en une dose d'environ 5 mg d'oxypurinol par kg de poids du corps du patient.

8. Utilisation de l'oxypurinol suivant l'une quelconque des revendications 1 à 4, sous la forme du sel de sodium.